# FASCICULE DE BREVET EUROPEEN

(11) **EP 0 985 402 B1**
(45) Date de publication et mention de la délivrance du brevet: **29.08.2001**
(21) Numéro de dépôt: 99401883.6
(22) Date de dépôt: 23.07.1999
(51) Int. Cl.: A61K 7/00, A61K 7/48, B01F 17/00

(54) **Emulsion H/E/H stable et son utilisation comme composition cosmétique et/ou dermatologique**
Stabile w/ö/w-Emulsion und ihre Verwendung als kosmetische und/oder dermatologische Zusammenstellung
Stable w/o/w emulsion and its use for cosmetic and/or dermatologic composition

(30) Priorité: 09.09.1998 FR 9811263
(43) Date de publication de la demande: 15.03.2000
(73) Titulaire: L'OREAL, 75008 Paris (FR)
(72) Inventeur: Simon, Pascal, 94400 Vitry/s/Seine (FR)
(74) Mandataire: Rasson, Catherine

(56) Documents cités:
- EP-A- 0 559 013

## Description

La présente invention se rapporte à une émulsion triple huile/eau/huile stable et à son utilisation notamment dans les domaines cosmétique et/ou dermatologique, en particulier pour la libération contrôlée d'actifs, en vue notamment de nettoyer, traiter, protéger et/ou hydrater la peau et/ou les muqueuses et/ou les fibres kératiniques et plus particulièrement en vue de traiter la peau sèche.

Il est connu d'utiliser, notamment dans les domaines cosmétique et dermatologique, des compositions topiques sous forme d'émulsions. Ces émulsions sont généralement des émulsions huile-dans-eau (H/E) ou eau-dans-huile (E/H). Il peut s'agir aussi d'émulsions multiples du type eau/huile/eau (E/H/E) ou huile/eau/huile (H/E/H). Parmi les émulsions multiples, on utilise de préférence les émulsions à phase aqueuse externe, à savoir les émulsions E/H/E, qui allient les avantages de la fraîcheur à l'application, apportée par l'eau présente dans la phase externe aqueuse, et du confort apporté par une quantité relativement importante d'huile.

Toutefois, les émulsions H/E/H présentent aussi de l'intérêt du fait de leur phase continue huileuse permettant de former à la surface de la peau un film lipidique qui prévient la perte d'eau transépidermique et protège la peau des agressions extérieures. En outre, on peut éviter l'effet gras de telles émulsions en y incorporant de nouvelles huiles légères et au toucher non gras comme par exemple les huiles de silicones de basse viscosité, certains esters d'acides gras et d'alcools gras à chaîne courte. Il est donc intéressant de disposer d'émulsions multiples de type H/E/H.

Toutefois, les émulsions multiples de manière générale sont peu exploitées car elles présentent fréquemment des problèmes de stabilité dans le temps. Le mécanisme de déstabilisation le plus fréquemment rencontré est la migration d'huile des gouttelettes internes vers le milieu huileux externe à travers la couche aqueuse intermédiaire, soit par simple diffusion d'huile à travers la membrane aqueuse, soit par rupture préalable du film aqueux provoquant la coalescence des gouttelettes d'huile internes et conduisant à un relargage d'huile dans le milieu huileux externe. Généralement ce phénomène dit de perte du caractère multiple finit par aboutir à un déphasage visible macroscopiquement et à l'obtention d'une émulsion H/E simple instable au lieu d'une émulsion triple.

Aussi, différents moyens ont été envisagés pour pallier à cet inconvénient. En particulier, une des solutions consiste à introduire dans la phase huileuse interne ou externe un ou plusieurs polymère(s) gélifiant(s) dont le rôle est de limiter durablement les mouvements d'huile de la phase interne vers la phase externe. Toutefois, les polymères capables de gélifier les huiles sont peu répandus et n'ont pas de bonnes propriétés cosmétiques : ils accentuent la sensation de gras et sont collants pendant et après l'application sur la peau. Les émulsions multiples obtenues présentent le défaut d'être collantes et longues à pénétrer dans la peau.

Il subsiste donc le besoin d'une émulsion multiple H/E/H stable n'ayant pas les inconvénients de celles de l'art antérieur et étant notamment agréables à utiliser sur la peau.

La demanderesse a maintenant trouvé de manière inattendue que l'utilisation d'un organopolysiloxane élastomère partiellement ou totalement réticulé comportant une chaîne polyoxyéthyléné et/ou polyoxypropyléné permettait l'obtention d'une émulsion multiple H/E/H stable sans nécessiter l'ajout d'autres agents stabilisants.

Aussi, la présente invention a pour objet une émulsion triple huile/eau/huile comportant une émulsion primaire huile-dans-eau et une phase huileuse externe, caractérisée en ce que l'émulsion triple contient au moins un organopolysiloxane élastomère partiellement ou totalement réticulé comportant une chaîne polyoxyéthylénée et/ou polyoxypropylénée.

La présente invention a aussi pour objet l'utilisation d'au moins un organopolysiloxane élastomère partiellement ou totalement réticulé comportant une chaîne polyoxyéthylénée et/ou polyoxypropylénée pour la stabilisation d'une émulsion triple huile/eau/huile.

L'émulsion triple selon l'invention a l'avantage d'être stable et de pouvoir notamment préserver l'activité d'actifs, notamment d'actifs lipophiles présents dans la phase huileuse interne, d'où ils sont libérés lors de l'application de la composition sur la peau, les muqueuses et/ou les cheveux.

Les organopolysiloxanes élastomères partiellement ou totalement réticulés utilisables dans l'émulsion selon la présente invention peuvent être introduits dans l'une ou l'autre des phases huileuses. Ils sont de préférence introduits dans la phase huileuse externe de l'émulsion. Ce sont généralement des émulsifiants. Ils peuvent être choisis notamment parmi les polymères réticulés décrits dans la demande EP-A-0545002. Ces organopolysiloxanes sont obtenus par polymérisation d'addition des composés (I) et (ll) suivants :
(I) un organohydrogènepolysiloxane de formule (1) :

   R¹ ₐR² _{b}H_{c}SiO_{(4-a-b-c)/2} (1)

   dans laquelle R¹ représente un groupe alkyle, aryle ou aralkyle substitué ou non substitué, comportant de 1 à 18 atomes de carbone, ou un groupe hydrocarboné halogéné ; R² représente un groupe :

   -CₙH₂ₙO(C₂H₄O)_{d}(C₃H₆O)ₑR³ (3)

   dans lequel R³ est un hydrogène, un groupe hydrocarboné aliphatique saturé ayant de 1 à 10 atomes de carbone ou un groupe -(CO)-R⁵ où R⁵ est groupe hydrocarboné aliphatique saturé ayant de 1 à 5 atomes de carbone; d est un nombre entier de 2 à 200 et e est un nombre entier de 0 à 200, pourvu que d + e soit un nombre allant de 3 à 200, et n est un nombre de 2 à 6, a est une valeur satisfaisant à l'inégalité : 1.0 ≤ a ≤ 2.5, b est une valeur satisfaisant à l'inégalité 0.001 ≤ b ≤ 1.0 et c est une valeur satisfaisant à l'inégalité : 0.001 ≤ c ≤ 1.0 ;
   ou un organohydrogènepolysiloxane représenté par la formule (2) suivante :

   R¹ _{f}H_{g}SiO_{(4-f-g)/2} (2)

   dans laquelle R¹ a la même signification que dans la formule (1), f est une valeur satisfaisant à l'inégalité : 1.0 ≤ f ≤ 3.0, g est une valeur satisfaisant à l'inégalité 0.001 ≤ g ≤1.5;
   ou un mélange des organohydrogènepolysiloxanes de formules (1) et (2), et
(II) un polyoxyalkylène représenté par la formule (A) suivante :

   CₘH₂ₘO(C₂H₄O)ₕ(C₃H₆O)ᵢCₘH₂ₘ₋₁ (A)

   dans laquelle h est un nombre entier allant de 2 à 200, i est un nombre entier allant de 0 à 200, pourvu que h + i soit un nombre allant de 3 à 200, et m est un nombre allant de 2 à 6,
   ou un organopolysiloxane représenté par la formule (B) suivante :

   R¹ ⱼR⁴ ₖSiO_{(4-j-k)/2} (B)

   dans laquelle R¹ a la même signification que dans la formule (1), R⁴ est un groupe hydrocarboné monovalent ayant une liaison aliphatique insaturée en extrémité et contenant 2 à 10 atomes de carbone, j est une valeur satisfaisant à l'inégalité : 1.0 ≤ j ≤ 3.0 et k est une valeur satisfaisant à l'inégalité : 0.001 ≤ k ≤ 1.5,
   ou un mélange du polyoxyalkylène de formule (A) et de l'organopolysiloxane de formule (B), où au moins un organohydrogènepolysiloxane de formule (1) ou au moins un polyoxyalkylène de formule (A) est contenu comme élément essentiel de la polymérisation d'addition.

De manière préférée, l'organopolysiloxane est en mélange avec une huile de silicone et/ou un polyol et est préparé directement en un tel mélange. L'huile de silicone présente de préférence une viscosité égale ou inférieure à 100 cSt à 25°C. Selon un mode de réalisation de l'invention, l'organopolysiloxane élastomère est préparé à partir de 100 parties en poids des constituants définis ci-dessus et 3 à 200 parties en poids d'une huile de silicone ayant une viscosité égale ou inférieure à 100 cSt à 25°C, et/ou d'un polyol. L'huile de silicone peut être une huile de silicone volatile ou non volatile ou un mélange d'huile de silicone volatile et d'huile de silicone non volatile.

Comme organopolysiloxane partiellement ou totalement réticulé comportant une chaîne polyoxyéthylénée et/ou polyoxypropylénée, on peut citer par exemple le produit commercialisé par Shin-Etsu sous la dénomination de KSG 21. Ce produit comprend 28 % d'organopolysiloxane et 62 % d'huile de silicone ayant une viscosité de 6 cSt.

Dans l'émulsion triple selon l'invention, l'organopolysiloxane partiellement ou totalement réticulé est utilisé de préférence en une quantité en matière active allant de 0,1 à 10 % et de préférence de 1 à 5 % en poids par rapport au poids total de l'émulsion triple.

De préférence, l'émulsion primaire H/E contient un ou plusieurs émulsifiants choisis parmi :
(1) les tensioactifs non ioniques ayant un HLB supérieur ou égal à 11, éventuellement associés à un agent co-tensioactif lipophile tel qu'un alcool gras, un acide gras ou un ester gras de glycéryle (stéarate de glycéryle). Comme tensioactif non ionique ayant un HLB supérieur ou égal à 11, on peut citer par exemple les esters d'acides gras et de glycérol oxyéthylénés, les esters d'acides gras et de sorbitan oxyéthylénés, les acides gras oxyéthylénés, les esters de sucres comme le stéarate de sucrose, et leurs mélanges ;
(2) les polymères susceptibles de stabiliser une émulsion H/E. Comme polymères de ce type, on peut citer par exemple les copolymères constitués d'une fraction majoritaire de monomère acide carboxylique monooléfiniquement insaturé en C₃-C₆ ou de son anhydride et d'une fraction minoritaire de monomère ester gras d'acide acrylique, tels que le produit commercialisé sous la dénomination Pemulen TR2 par la société Goodrich (nom CTFA : acrylates / C10-30 alkyl acrylate crosspolymer), ou les polyacrylamides tels que le produit commercialisé sous la dénomination Hostacerin AMPS par la société Hoechst (nom CTFA : Ammonium polyacryldimethyltauramide), et leurs mélanges ;
(3) les dispersions de vésicules lipidiques à base de lipides amphiphiles ioniques (liposomes) ou non ioniques, et notamment les liposomes à base de l'association lécithine hydrogénée / stérol de soja oxyéthyléné.

On peut aussi utiliser un mélange de ces émulsifiants.

Ces émulsifiants peuvent être introduits dans la phase aqueuse ou dans la phase huileuse de l'émulsion primaire.

La quantité d'émulsifiant(s) dans l'émulsion primaire varie selon la nature des émulsifiants utilisés. Elle peut aller par exemple de 0,1 à 15 % en poids par rapport au poids total de l'émulsion primaire.

La quantité d'émulsion primaire H/E dans l'émulsion triple va généralement de 50 à 95 % et de préférence de 70 à 85 % en poids par rapport au poids total de l'émulsion triple.

La quantité de la phase huileuse interne va généralement de 0,1 à 40 % et de préférence 1 à 25 % en poids par rapport au poids total de l'émulsion triple. La quantité de la phase aqueuse va généralement de 10 à 90 % et de préférence 40 à 80 % en poids par rapport au poids total de l'émulsion triple.

La phase huileuse de l'émulsion primaire H/E et la phase huileuse externe comprennent un ou plusieurs corps gras choisis parmi les huiles d'origine animale, les huiles d'origine végétale (huile d'amande d'abricot, fraction liquide de beurre de karité), les huiles minérales (vaseline), les huiles synthétiques (isohexadécane, polyisobutène hydrogéné ou huile de Parleam), les huiles fluorées, les huiles de silicone et notamment les huiles de silicone volatiles comme l'octyl-heptaméthyltrisiloxane (ou caprylylmethicone) et les cyclométhicones tels que le cyclopentasiloxane et le cyclohexasiloxane, les cires et notamment les cires de silicone, les gommes de silicone, les résines de silicone.

De manière préférée, la phase huileuse contenant l'organopolysiloxane élastomère et notamment la phase huileuse externe de l'émulsion multiple H/E/H, contient comme corps gras, au moins un solvant de l'organopolysiloxane élastomère, qui est de préférence une huile de silicone choisie parmi les organopolysiloxanes cycliques (cyclomethicone) tels que le cyclohexaméthylsiloxane ou les organopolysiloxanes linéaires de basse viscosité (polydiméthylsiloxanes de viscosité inférieure à 50 cSt ou dimethicones).

L'émulsion triple est préparée de manière classique par préparation de l'émulsion primaire et incorporation d'une quantité déterminée de l'émulsion primaire dans la phase huileuse externe qui selon un mode préféré de réalisation de l'invention, contient l'organopolysiloxane élastomère partiellement ou totalement réticulé comportant une chaîne polyoxyéthylénée et/ou polyoxypropylénée.

L'émulsion triple selon l'invention peut être utilisée notamment dans les domaines cosmétique, dermatologique et pharmaceutique. De préférence, elle est destinée à constituer une composition pour application topique. Dans ce cas, la composition doit contenir un milieu topiquement acceptable, c'est-à-dire compatible avec la peau, les muqueuses, les ongles, le cuir chevelu et/ou les cheveux. Comme indiqué en début de description, l'un des intérêts majeurs de l'émulsion conforme à l'invention est que cette dernière peut contenir, tout en présentant un caractère stable, des principes actifs tant cosmétiques que thérapeutiques, ces actifs pouvant donc être notamment choisis parmi tous ceux habituellement utilisés à ce jour dans le domaine de la cosmétique, de la dermatologie ou du médicament.

Cet actif peut être notamment un actif lipophile, mais il peut être aussi hydrophile, et, selon sa nature, il peut être introduit dans une des phases huileuses ou dans la phase aqueuse de l'émulsion triple selon l'invention.

Comme actifs lipophiles, on peut citer notamment les vitamines lipophiles telles que la vitamine A (rétinol), la vitamine D, la vitamine E (tocophérol), la vitamine K et les dérivés de ces vitamines tels que les esters, les céramides, les insaponifiables tels que l'insaponifiable de karité, les extraits d'algues notamment ceux riches en acides gras polyinsaturés tels que l'acide eicosa-pentaènoïque et l'acide docosa-hexaènoïque, les huiles insaturées par exemple les huiles de poisson riches en acides linoléiques et linoléniques.

Comme actifs hydrophiles, on peut citer notamment les polyols tels que la glycérine, les glycols et les dérivés de sucre, les enzymes, les extraits naturels, les oligomères procyannidoliques, les vitamines telles que la vitamine C et ses dérivés tels que les esters, l'urée, les dépigmentants tels que l'acide kojique et l'acide caféique, les bêta-hydroxyacides tels que l'acide salicylique et ses dérivés, les alpha-hydroxyacides tels que l'acide lactique et l'acide glycolique, les hydratants tels que les hydrolysats de protéines, les adoucissants tels que l'allantoïne et leurs mélanges.

L'actif est présent en une quantité efficace pour assurer le résultat escompté. Il peut être par exemple présent dans l'émulsion selon l'invention en une quantité allant de 0,01 à 20 %, de préférence de 0,1 à 10 % et mieux de 0,5 à 5 % en poids par rapport au poids total de la composition.

Les émulsions H/E/H selon l'invention peuvent constituer notamment des compositions de nettoyage, de protection, de traitement et/ou de soin de la peau, des muqueuses et/ou des cheveux, en particulier pour le visage, pour le cou, pour les mains, pour les cheveux, pour le cuir chevelu ou pour le corps, ainsi que pour les cils. Elles peuvent constituer notamment des crèmes de protection, de traitement ou de soin pour le visage, pour les mains, pour les pieds, des laits corporels de protection ou de soin, des lotions, gels ou mousses pour le soin de la peau, des muqueuses, des cheveux et du cuir chevelu. Les compositions de l'invention sont particulièrement appropriées pour hydrater la peau et/ou traiter la peau sèche.

Aussi, l'invention a encore pour objet l'utilisation cosmétique de l'émulsion telle que définie ci-dessus pour nettoyer et/ou traiter et/ou protéger et/ou hydrater la peau et/ou les muqueuses et/ou les fibres kératiniques, c'est-à-dire les cheveux et/ou les cils.

L'invention a aussi pour objet l'utilisation de l'émulsion telle que définie ci-dessus pour la préparation d'une composition dermatologique destinée à traiter et/ou protéger la peau sèche.

L'invention a aussi pour objet un procédé cosmétique et/ou dermatologique pour nettoyer et/ou traiter et/ou protéger et/ou hydrater la peau, les muqueuses et/ou les fibres kératiniques, caractérisé en ce qu'il consiste à appliquer sur la peau, les muqueuses et/ou les fibres kératiniques, une émulsion telle que définie ci-dessus.

De façon connue, la composition de l'invention peut contenir également des adjuvants lipophiles ou hydrophiles habituels dans les domaines cosmétique et dermatologique, tels que les tensioactifs moussants, les conservateurs, les antioxydants, les séquestrants, les solvants (octyldodécanol), les parfums, les charges, les filtres solaires, les absorbeurs d'odeur, les matières colorantes, les gélifiants et les vésicules lipidiques.

Comme gélifiants, on peut citer par exemple les argiles, les gommes polysaccharides et leurs dérivés (gomme de xanthane, carboxyméthylhydroxypropyl guar), les polymères carboxyvinyliques (carbomer ou sodium carbomer).

Les quantités de ces différents adjuvants sont celles classiquement utilisées dans les domaines considérés, et par exemple de 0,01 à 15 % du poids total de la composition. La nature des adjuvants et leurs quantités doivent être telles qu'elles ne modifient pas les propriétés des compositions selon l'invention.

Les adjuvants, selon leur nature, peuvent être introduits dans la phase aqueuse ou dans une des phases huileuses de l'émulsion triple.

Les exemples ci-après de compositions selon l'invention, sont donnés à titre d'illustration. Les quantités y sont données en % en poids par rapport au poids total de la composition.

### Exemple 1 : crème de nuit hydratante

### 1. Emulsion primaire H/E :

### Phase A : Préparation de liposomes

- Lécithine hydrogénée (Lécinol S 10 commercialisé par NIKKOL) 0,72 %
- Stérols de soja oxyéthylénés (Generol 122 N E 5D commercialisé par HENKEL) 2,8 %
- Eau 9 %

### Phase B : phase huileuse de l'émulsion primaire H/E

- Isohexadécane 3,5 %
- Huile d'amande d'abricot 5 %
- Insaponifiables de karité 1 %
- Parfum 0,5 %
- Conservateur 0,6 %

### Phase C : phase aqueuse

- Glycérine 3 %
- conservateur 0,2 %
- Gomme de xanthane 0,2 %
- Sodium carbomer 0,15 %
- Eau 53,33 %

### 2. Phase huileuse externe :

- Cyclohexasiloxane 12 %
- KSG 21 à 28% de matière active 8 %

### Exemple 2 : crème nourrissante pour peaux sèches

### 1. Emulsion primaire H/E :

### Phase A :

- Glycéryl stearate and PEG-100 glycéryl stearate (Arlacel 165 commercialisé ICI) 1,8 %
- Polysorbate 60 (Tween 60 commercialisé par ICI) 0,75 %
- Fraction liquide de beurre de karité 7,3 %
- Alcool cétylique 1,75 %
- Cyclopentasiloxane (DC Fluid 245 commercialisé par Dow Corning) 7 %
- Huile de parleam 4 %
- Parfum 0,5 %
- Conservateur 0,6 %

### Phase B

- Glycérine 7 %
- conservateur 0,2 %
- Gomme de xanthane 0,1 %
- Sodium carbomer 0,2 %
- Eau 53,8 %

### 2. Phase huileuse externe :

- Dimethicone 10 cSt (DC Fluid 200 commercialisé par Dow Corning) 10 %
- KSG 21 à 28% de matière active 8 %

### Exemple 3 : crème hydratante

### 1. Emulsion primaire H/E:

### Phase A :

- Octyldodécanol 2 %
- Cyclopentasiloxane (DC Fluid 245 commercialisé par Dow Corning) 4,5 %
- Acétate de tocophéryle 1 %
- Parfum 0,5 %
- Conservateur 0,6 %

### Phase B :

- Glycérine 5,5 %
- conservateur 0,2 %
- Acrylate/C 10-30 alkyl acrylate crosspolymer
   (Pemulen TR 2 commercialisé par GOODRICH) 0,13 %
- Ammonium polyacryldimethyltauramide
   (Hostacerin AMPS commercialisé par HOECHST) 1,1 %
- Eau 69,47 %

### 2. Phase huileuse externe :

- Caprylylmethicone (Silsoft 034 commercialisé par WITCO) 7 %
- KSG 21 à 28% de matière active 8 %

## Revendications

1. Emulsion triple huile/eau/huile comportant une émulsion primaire huite-dans-eau et une phase huileuse externe, **caractérisée en ce que** l'émulsion triple contient au moins un organopolysiloxane élastomère partiellement ou totalement réticulé comportant une chaîne polyoxyéthylénée et/ou polyoxypropylénée, le dit organopolyosiloxane élastomère étant obtenu par polymérisation d'addition des composés (I) et (II) suivants :
(I) un organohydrogènepolysiloxane de formule (1) :
R¹ ₐR² _{b}H_{c}SiO_{(4-a-b-c)/2} (1)
dans laquelle R¹ représente un groupe alkyle, aryle ou aralkyle substitué ou non substitué, comportant de 1 à 18 atomes de carbone, ou un groupe hydrocarboné halogéné ; R² représente un groupe :
-CₙH₂ₙO(C₂H₄O)_{d}(C₃H₆O)ₑR³ (3)
dans lequel R³ est un hydrogène, un groupe hydrocarboné aliphatique saturé ayant de 1 à 10 atomes de carbone ou un groupe -(CO)-R⁵ où R⁵ est groupe hydrocarboné aliphatique saturé ayant de 1 à 5 atomes de carbone; d est un nombre entier de 2 à 200 et e est un nombre entier de 0 à 200, pourvu que d + e soit un nombre allant de 3 à 200, et n est un nombre de 2 à 6, a est une valeur satisfaisant à l'inégalité : 1.0 ≤ a ≤ 2.5, b est une valeur satisfaisant à l'inégalité : 0.001 ≤ b ≤ 1.0 et c est une valeur satisfaisant à l'inégalité : 0.001 ≤ c ≤ 1.0 ;
ou un organohydrogènepolysiloxane représenté par la formule (2) suivante :
R¹ _{f}H_{g}SiO_{(4-f-g)/2} (2)
dans laquelle R¹ a la même signification que dans la formule (1), f est une valeur satisfaisant à l'inégalité : 1.0 ≤ f ≤ 3.0, g est une valeur satisfaisant à l'inégalité : 0.001 ≤ g ≤ 1.5;
ou un mélange des organohydrogènepolysiloxanes de formules (1) et (2), et
(II) un polyoxyalkylène représenté par la formule (A) suivante :
CₘH₂ₘO(C₂H₄O)ₕ(C₃H₈O)ᵢCₘH₂ₘ₋₁ (A)
dans laquelle h est un nombre entier allant de 2 à 200, i est un nombre entier allant de 0 à 200, pourvu que h + i soit un nombre allant de 3 à 200, et m est un nombre allant de 2 à 6,
ou un organopolysiloxane représenté par la formule (B) suivante :
R¹ ⱼR⁴ ₖSiO_{(4-j-k)/2} (B)
dans laquelle R¹ a la même signification que dans la formule (1), R⁴ est un groupe hydrocarboné monovalent ayant une liaison aliphatique insaturée en extrémité et contenant 2 à 10 atomes de carbone, j est une valeur satisfaisant à l'inégalité : 1.0 ≤ j ≤ 3.0 et k est une valeur satisfaisant à l'inégalité : 0.001 ≤ k ≤ 1.5,
ou un mélange du polyoxyalkylène de formule (A) et de l'organopolysiloxane de formule (B), où au moins un organohydrogènepolysiloxane de formule (1) ou au moins un polyoxyalkylène de formule (A) est contenu comme élément essentiel de la polymérisation d'addition.

2. Emulsion selon la revendication 1, caractérisée par le fait que l'organopolysiloxane élastomère est présent dans la phase huileuse externe.

3. Emulsion selon la revendication 1 ou 2, caractérisée par le fait que l'organopolysiloxane élastomère est en mélange avec une huile de silicone et/ou un polyol.

4. Emulsion selon la revendication précédente, caractérisée par le fait que l'huile de silicone a de préférence une viscosité égale ou inférieure à 100 cSt à 25°C.

5. Emulsion selon la revendication 3 ou 4, caractérisée par le fait que l'huile de silicone a une viscosité égale à 6 cSt à 25°C.

6. Emulsion selon l'une quelconque des revendications précédentes, **caractérisée en ce que** l'organopolysiloxane élastomère est présent en une quantité en matière active allant de 0,1 à 10 % en poids par rapport au poids total de l'émulsion triple.

7. Emulsion selon l'une quelconque des revendications précédentes, **caractérisée en ce que** l'émulsion primaire H/E contient un ou plusieurs émulsifiants choisis parmi les tensioactifs non ioniques ayant un HLB supérieur ou égal à 11, les polymères susceptibles de stabiliser une émulsion H/E, les dispersions de vésicules lipidiques et leurs mélanges.

8. Emulsion selon la revendication précédente, **caractérisée en ce que** le tensioactif non ionique est choisi parmi les esters d'acides gras et de glycérol oxyéthylénés, les esters d'acide gras et de sorbitane oxyéthylénés, les acides gras oxyéthylénés, les esters de sucres et leurs mélanges.

9. Emulsion selon la revendication 7, **caractérisée en ce que** le polymère est choisi parmi les copolymères constitués d'une fraction majoritaire de monomère acide carboxylique monooléfiniquement insaturé en C₃-C₆ ou de son anhydride et d'une fraction minoritaire de monomère ester gras d'acide acrylique, les polyacrylamides et leurs mélanges.

10. Emulsion selon la revendication 7, **caractérisée en ce que** la dispersion de vésicules lipidiques est une dispersion de liposomes.

11. Emulsion selon l'une quelconque des revendications précédentes, **caractérisée en ce que** les phases huileuses contiennent un ou plusieurs corps gras choisis parmi les huiles d'origine animale, les huiles d'origine végétale, les huiles minérales, les huiles synthétiques, les huiles de silicone, les huiles fluorées, les cires, les gommes de silicone, les résines de silicone.

12. Emulsion selon l'une quelconque des revendications précédentes, **caractérisée en ce que** la quantité de phase huileuse interne va de 0,1 à 40 % et de préférence 1 à 25 % en poids par rapport au poids total de l'émulsion triple.

13. Emulsion selon l'une quelconque des revendications précédentes, **caractérisée en ce que** la quantité d'émulsion primaire H/E va de 50 à 95 % et de préférence de 70 à 85 % en poids par rapport au poids total de l'émulsion triple.

14. Emulsion selon l'une quelconque des revendications précédentes, **caractérisée en ce qu**'elle constitue une composition pour application topique.

15. Emulsion selon l'une quelconque des revendications précédentes, **caractérisée en ce qu**'elle contient au moins un actif choisi parmi les vitamines, les céramides, les insaponifiables, les extraits d'algues, les huiles insaturées, les polyols, les enzymes, les extraits naturels, les oligomères procyannidoliques, l'urée, les dépigmentants, les bêta-hydroxyacides, les alpha-hydroxyacides, les hydratants, les adoucissants et leurs mélanges.

16. Emulsion selon la revendication précédente, **caractérisée en ce que** la quantité d'actif(s) va de 0,01 à 20 % et de préférence de 0,1 à 10 % en poids par rapport au poids total de l'émulsion triple.

17. Emulsion selon l'une quelconque des revendications précédentes, **caractérisée en ce qu**'elle comprend au moins un adjuvant lipophile ou hydrophile choisi parmi les conservateurs, les antioxydants, les séquestrants, les solvants, les parfums, les charges, les filtres solaires, les absorbeurs d'odeur, les matières colorantes, les gélifiants et les vésicules lipidiques.

18. Utilisation cosmétique de l'émulsion selon l'une quelconque des revendications précédentes pour nettoyer et/ou traiter et/ou protéger et/ou hydrater la peau, les muqueuses et/ou les fibres kératiniques, les méthodes de traitement thérapeutiques du corps humain étant exclues.

19. Utilisation de l'émulsion selon l'une quelconque des revendications 1 à 17 pour la préparation d'une composition dermatologique destinée à traiter et/ou protéger la peau sèche.

20. Procédé cosmétique pour nettoyer et/ou traiter et/ou protéger et/ou hydrater la peau et/ou les fibres kératiniques, **caractérisé en ce qu**'il consiste à appliquer sur la peau, les muqueuses et/ou les fibres kératiniques, une émulsion selon l'une quelconque des revendications 1 à 17, les méthodes de traitement thérapeutiques du corps humain étant exclues.

21. Utilisation d'au moins un organopolysiloxane élastomère partiellement ou totalement réticulé comportant une chaîne polyoxyéthylénée et/ou polyoxypropylénée, pour la stabilisation d'une émulsion triple huile/eau/huile.

## Patentansprüche

1. Dreifache Ö1/Wasser/Öl-Emulsion, die eine primäre Öl-in-Wasser-Emulsion und eine äußere Ölphase aufweist, **dadurch gekennzeichnet**, dass die dreifache Emulsion mindestens ein teilweise oder vollständig vernetztes elastomeres Polyorganosiloxan enthält, das eine polyethoxylierte und/oder polypropoxylierte Kette aufweist, wobei das elastomere Polyorganosiloxan durch Additionspolymerisation der folgenden Verbindungen (I) und (II) hergestellt wird:
(I) eines Polyorganohydrogenosiloxans der Formel (1):
R¹ ₐR² _{b}H_{c}SiO_{(4-a-b-c)/2} (1)
worin bedeuten:
- R¹ eine Alkylgruppe, eine Arylgruppe oder eine Aralkylgruppe, die substituiert oder nicht substituiert ist und 1 bis 18 Kohlenstoffatome aufweist, oder eine halogenierte Kohlenwasserstoffgruppe,
- R² eine Gruppe:
-CₙH₂ₙO(C₂H₄O)_{d}(C₃H₆O)ₑR³ (3)
worin bedeuten:
- R³ Wasserstoff, eine gesättigte aliphatische Kohlenwasserstoffgruppe mit 1 bis 10 Kohlenstoffatomen oder eine Gruppe-(CO)-R⁵, worin R⁵ eine gesättigte aliphatische Kohlenwasserstoffgruppe mit 1 bis 5 Kohlenstoffatomen ist,
- d eine ganze Zahl von 2 bis 200 und e Null oder eine ganze Zahl von 1 bis 200, mit der Maßgabe, dass d + e eine Zahl im Bereich von 3 bis 200 ist, und
- n eine Zahl von 2 bis 6,
- a einen Wert, der die folgende Beziehung erfüllt: 1,0 ≤ a ≤ 2,5,
- b einen Wert, der die folgende Beziehung erfüllt: 0,001 ≤ b ≤ 1,0, und
- c einen Wert, der die folgende Beziehung erfüllt: 0,001 ≤ c ≤ 1,0,
oder eines Polyorganohydrogenosiloxans der folgenden Formel (2):
R¹ _{f}H_{g}SiO_{(4-f-g)/2} (2)
worin:
- R¹ die in Formel (1) angegebene Bedeutung aufweist,
- f einen Wert bedeutet, der die folgende Beziehung erfüllt: 1,0 ≤ f ≤ 3,0, und
- g einen Wert bedeutet, der die folgende Beziehung erfüllt: 0,001 ≤ g ≤ 1,5,
oder eines Gemisches der Polyorganohydrogenosiloxane der Formeln (1) und (2) und
(II) eines Polyalkylenoxids der folgenden Formel (A):
CₘH₂ₘO(C₂H₄O)ₕ(C₃H₆O)ᵢCₘH₂ₘ₋₁ (A)
worin bedeuten:
- h eine ganze Zahl im Bereich von 2 bis 200 und i Null oder eine ganze Zahl im Bereich von 1 bis 200, mit der Maßgabe, dass h + i eine Zahl im Bereich von 3 bis 200 ist, und
- m eine Zahl im Bereich von 2 bis 6,
oder eines Polyorganosiloxans der folgenden Formel (B):
R¹ ⱼR⁴ ₖSiO_{(4-j-k)/2} (B)
worin:
- R¹ die in Formel (1) angegebene Bedeutung aufweist,
- R⁴ eine einwertige Kohlenwasserstoffgruppe mit einer endständigen ungesättigten aliphatischen Bindung bedeutet, die 2 bis 10 Kohlenstoffatome enthält,
- j einen Wert bedeutet, der die folgende Beziehung erfüllt: 1,0 ≤ j ≤ 3,0, und
- k einen Wert bedeutet, der die folgende Beziehung erfüllt: 0,001 ≤ k ≤ 1,5,
oder eines Gemisches des Polyalkylenoxids der Formel (A) und des Polyorganosiloxans der Formel (B),
wobei mindestens ein Polyorganohydrogenosiloxan der Formel (1) oder mindestens ein Polyalkylenoxid der Formel (A) als wesentliches Element der Additionspolymerisation enthalten ist.

2. Emulsion nach Anspruch 1, **dadurch gekennzeichnet**, dass das elastomere Polyorganosiloxan in der äußeren Ölphase vorliegt.

3. Emulsion nach Anspruch 1 oder 2, **dadurch gekennzeichnet**, dass das elastomere Polyorganosiloxan im Gemisch mit einem Siliconöl und/oder einem Polyol vorliegt.

4. Emulsion nach dem vorhergehenden Anspruch, **dadurch gekennzeichnet** dass das Siliconöl bei 25 °C vorzugsweise eine Viskosität von kleiner oder gleich 100 cSt aufweist.

5. Emulsion nach Anspruch 3 oder 4, **dadurch gekennzeichnet**, dass das Siliconöl bei 25 °C eine Viskosität von 6 cSt aufweist.

6. Emulsion nach einem der vorhergehenden Ansprüche, **dadurch gekennzeichnet**, dass das elastomere Polyorganosiloxan in einem als Wirkstoff ausgedrückten Mengenanteil von 0,1 bis 10 Gew.-%, bezogen auf das Gesamtgewicht der dreifachen Emulsion, vorliegt.

7. Emulsion nach einem der vorhergehenden Ansprüche, **dadurch gekennzeichnet**, dass die primäre O/W-Emulsion einen oder mehrere Emulgatoren enthält, die unter nichtionischen grenzflächenaktiven Stoffen mit einem HLB-Wert von größer oder gleich 11, Polymeren, die zur Stabilisierung einer O/W-Emulsion befähigt sind, Dispersionen von Lipidvesikeln und deren Gemischen ausgewählt sind.

8. Emulsion nach dem vorhergehenden Anspruch, **dadurch gekennzeichnet**, dass der nichtionische grenzflächenaktive Stoff unter ethoxylierten Estern von Fettsäuren und Glycerin, ethoxylierten Estern von Fettsäuren und Sorbitan, ethoxylierten Fettsäuren, Estern von Zuckern und den Gemische dieser Verbindungen ausgewählt ist.

9. Emulsion nach Anspruch 7, **dadurch gekennzeichnet**, dass das Polymer unter Copolymeren, die aus monoolefinisch ungesättigten Carbonsäuremonomeren mit 3 bis 6 Kohlenstoffatomen oder ihren Anhydriden als Hauptanteil und in geringerem Anteil Acrylfettestermonomeren bestehen, Polyacrylamiden und den Gemischen dieser Verbindungen ausgewählt ist.

10. Emulsion nach Anspruch 7, **dadurch gekennzeichnet**, dass die Dispersion von Lipidvesikeln eine Dispersion von Liposomen ist.

11. Emulsion nach einem der vorhergehenden Ansprüche, **dadurch gekennzeichnet**, dass die Ölphasen eine oder mehrere Fettsubstanzen enthalten, die unter Ölen tierischen Ursprungs, Ölen pflanzlichen Ursprungs, Mineralölen, synthetischen Ölen, Siliconölen, fluorierten Ölen, Wachsen, Silicongummis und Siliconharzen ausgewählt sind.

12. Emulsion nach einem der vorhergehenden Ansprüche, **dadurch gekennzeichnet**, dass der Mengenanteil der inneren Ölphase im Bereich von 0,1 bis 40 Gew.-% und vorzugsweise von 1 bis 25 Gew.-%, bezogen auf das Gesamtgewicht der dreifachen Emulsion, liegt.

13. Emulsion nach einem der vorhergehenden Ansprüche, **dadurch gekennzeichnet**, dass der Mengenanteil der primären O/W-Emulaion im Bereich von 50 bis 95 Gew.-% und vorzugsweise von 70 bis 85 Gew.-%, bezogen auf das Gesamtgewicht der dreifachen Emulsion, liegt.

14. Emulsion nach einem der vorhergehenden Ansprüche, **dadurch gekennzeichnet**, dass sie eine Zusammensetzung zur topischen Anwendung darstellt.

15. Emulsion nach einem der vorhergehenden Ansprüche, **dadurch gekennzeichnet**, dass sie mindestens einen Wirkstoff enthält, der unter Vitaminen, Ceramiden, nicht verseifbaren Verbindungen, Algenextrakten, ungesättigten Ölen, Polyolen, Enzymen, natürlichen Extrakten, Procyannidol-Oligomeren, Harnstoff, depigmentierenden Stoffen, β-Hydroxysäuren, α-Hydroxysäuren, hydratisierenden Stoffen, reizlindernden Stoffen und deren Gemischen ausgewählt ist.

16. Emulsion nach dem vorhergehenden Anspruch, **dadurch gekennzeichnet**, dass der Mengenanteil des Wirkstoffs bzw. der Wirkstoffe im Bereich von 0,01 bis 20 Gew.-% und vorzugsweise von 0,1 bis 10 Gew.-%, bezogen auf das Gesamtgewicht der dreifachen Emulsion, liegt.

17. Emulsion nach einem der vorhergehenden Ansprüche, **dadurch gekennzeichnet**, dass sie mindestens einen lipophilen oder hydrophilen Zusatzstoff enthält, der unter Konservierungsstoffen, Antioxidantien, Maskierungsmitteln, Lösemitteln, Parfums, Füllstoffen, Sonnenschutzfiltern, Geruchsabsorbern, Färbemitteln, Gelbildnern und Lipidvesikeln ausgewählt ist.

18. Kosmetische Verwendung der Emulsion nach einem der vorhergehenden Ansprüche zur Reinigung und/oder zur Behandlung und/ oder zum Schutz und/oder zur Hydratisierung der Haut, der Schleimhäute und/oder der Keratinfasern, wobei die therapeutischen Verfahren zur Behandlung des menschlichen Körpers ausgeschlossen sind.

19. Verwendung der Emulsion nach einem der Ansprüche 1 bis 17 zur Herstellung einer dermatologischen Zusammensetzung, die zur Behandlung und/oder zum Schutz trockener Haut bestimmt ist.

20. Kosmetisches Verfahren zur Reinigung und/oder zur Behandlung und/oder zum Schutz und/oder zur Hydratisierung der Haut und/ oder der Keratinfasern, **dadurch gekennzeichnet**, dass es darin besteht, eine Emulsion nach einem der Ansprüche 1 bis 17 auf die Haut, die Schleimhäute und/oder die Keratinfasern aufzubringen, wobei die therapeutischen Verfahren zur Behandlung des menschlichen Körpers ausgeschlossen sind.

21. Verwendung mindestens eines teilweise oder vollständig vernetzten elastomeren Polyorganosiloxans, das eine polyethoxylierte und/oder polypropoxylierte Kette aufweist, zur Stabilisierung einer dreifachen Ö1/Wasser/Ö1-Emulsion.

## Claims

1. Triple oil-water-oil emulsion comprising a primary oil-in-water emulsion and an outer oily phase, **characterized in that** the triple emulsion contains at least one partially or completely crosslinked elastomeric organopolysiloxane comprising a polyoxyethylenated and/or polyoxypropylenated chain, the said elastomeric organopolysiloxane being obtained by addition polymerization of the following compounds (I) and (II):
(I) an organohydrogenpolysiloxane of formula (1):
R¹ ₐR² _{b}H_{c}SiO_{(4-a-b-c)/2} (1)
in which R¹ represents a substituted or unsubstituted alkyl, aryl or aralkyl group, comprising from 1 to 18 carbon atoms, or a halogenated hydrocarbon group; R² represents a group:
-CₙH₂ₙO(C₂H₄O)_{d}(C₃H₆O)ₑR³ (3)
in which R³ is a hydrogen, a saturated aliphatic hydrocarbon group having from 1 to 10 carbon atoms or a group -(CO)-R⁵ where R⁵ is a saturated aliphatic hydrocarbon group having from 1 to 5 carbon atoms; d is an integer from 2 to 200 and e is an integer from 0 to 200, provided that d + e is a number ranging from 3 to 200, and n is a number from 2 to 6, a is a value satisfying the inequality: 1.0 ≤ a ≤ 2.5, b is a value satisfying the inequality: 0.001 ≤ b ≤ 1.0 and c is a value satisfying the inequality: 0.001 ≤ c ≤ 1.0;
or an organohydrogenpolysiloxane represented by the following formula (2):
R¹ _{f}H_{g}SiO_{(4-f-g)/2} (2)
in which R¹ has the same meaning as in formula (1), f is an integer satisfying the inequality: 1.0 ≤ f ≤ 3.0, g is a value satisfying the inequality: 0.001 ≤ g ≤ 1.5;
or a mixture of the organohydrogenpolysiloxanes of formulae (1) and (2), and
(II) a polyoxyalkylene represented by the following formula (A):
CₘH₂ₘO(C₂H₄O)ₕ(C₃H₆O)ᵢCₘH₂ₘ₋₁ (A)
in which h is an integer ranging from 2 to 200, i is an integer ranging from 0 to 200, provided that h + i is a number ranging from 3 to 200, m is a number ranging from 2 to 6,
or an organopolysiloxane represented by the following formula (B)
R¹ ⱼR⁴ ₖSiO_{(4-j-k)/2} (B)
in which R¹ has the same meaning as in formula (1), R⁴ is a monovalent hydrocarbon group having an unsaturated aliphatic bond at the end and containing 2 to 10 carbon atoms, j is a value satisfying the inequality: 1.0 ≤ j ≤ 3.0 and k is a value satisfying the inequality: 0.001 ≤ k ≤ 1.5,
or a mixture of the polyoxyalkylene of formula (A) and the organopolysiloxane of formula (B), where at least one organohydrogenpolysiloxane of formula (1) or at least one polyoxyalkylene of formula (A) is contained as an essential component of the addition polymerization.

2. Emulsion according to Claim 1, **characterized in that** the elastomeric organopolysiloxane is present in the outer oily phase.

3. Emulsion according to Claim 1 or 2, **characterized in that** the elastomeric organopolysiloxane is in the form of a mixture with a silicone oil and/or a polyol.

4. Emulsion according to the preceding claim, **characterized in that** the silicone oil preferably has a viscosity equal to or less than 100 cSt at 25°C.

5. Emulsion according to Claim 3 or 4, **characterized in that** the silicone oil has a viscosity equal to 6 cSt at 25°C.

6. Emulsion according to any one of the preceding claims, **characterized in that** the elastomeric organopolysiloxane is present in a quantity, as active substance, ranging from 0.1 to 10% by weight relative to the total weight of the triple emulsion.

7. Emulsion according to any one of the preceding claims, **characterized in that** the primary O/W emulsion contains one or more emulsifiers chosen from nonionic surfactants having an HLB greater than or equal to 11, polymers capable of stabilizing an O/W emulsion, dispersions of lipid vesicles and mixtures thereof.

8. Emulsion according to the preceding claim, **characterized in that** the nonionic surfactant is chosen from oxyethylenated esters of fatty acids and of glycerol, oxyethylenated esters of fatty acids and of sorbitan, oxyethylenated fatty acids, esters of sugars and mixtures thereof.

9. Emulsion according to Claim 7, **characterized in that** the polymer is chosen from copolymers consisting of a predominant fraction of a monoolefinically unsaturated C₃-C₆ carboxylic acid monomer or of its anhydride and a minor fraction of a fatty ester of acrylic acid monomer, polyacrylamides and mixtures thereof.

10. Emulsion according to Claim 7, **characterized in that** the dispersion of lipid vesicles is a dispersion of liposomes.

11. Emulsion according to any one of the preceding claims, **characterized in that** the oily phases contain one or more fatty substances chosen from oils of animal origin, oils of plant origin, mineral oils, synthetic oils, silicone oils, fluorinated oils, waxes, silicone gums, silicone resins.

12. Emulsion according to any one of the preceding claims, **characterized in that** the quantity of inner oily phase ranges from 0.1 to 40% and preferably 1 to 25% by weight relative to the total weight of the triple emulsion.

13. Emulsion according to any one of the preceding claims, **characterized in that** the quantity of primary O/W emulsion ranges from 50 to 95% and preferably from 70 to 85% by weight relative to the total weight of the triple emulsion.

14. Emulsion according to any one of the preceding claims, **characterized in that** it constitutes a composition for topical application.

15. Emulsion according to any one of the preceding claims, **characterized in that** it contains at least one active agent chosen from vitamins, ceramides, unsaponifiable components, extracts of algae, unsaturated oils, polyols, enzymes, natural extracts, procyannidolic oligomers, urea, depigmenting agents, beta-hydroxy acids, alpha-hydroxy acids, moisturizing agents, emollients and mixtures thereof.

16. Emulsion according to the preceding claim, **characterized in that** the quantity of active agent(s) ranges from 0.01 to 20% and preferably from 0.1 to 10% by weight relative to the total weight of the triple emulsion.

17. Emulsion according to any one of the preceding claims, **characterized in that** it comprises at least one lipophilic or hydrophilic adjuvant chosen from preservatives, antioxidants, sequestrants, solvents, perfumes, fillers, sunscreens, odour absorbers, colouring matter, gelling agents and lipid vesicles.

18. Cosmetic use of the emulsion according to any one of the preceding claims for cleansing and/or treating and/or protecting and/or moisturizing the skin, the mucous membranes and/or the keratinous fibres, the therapeutic methods of treating the human body being excluded.

19. Use of the emulsion according to any one of Claims 1 to 17 for the preparation of a dermatological composition intended for treating and/or protecting dry skin.

20. Cosmetic method for cleansing and/or treating and/or protecting and/or moisturizing the skin and/or the keratinous fibres, **characterized in that** it consists in applying to the skin, the mucous membranes and/or the keratinous fibres an emulsion according to any one of Claims 1 to 17, the therapeutic methods of treating the human body being excluded.

21. Use of at least one partially or completely crosslinked elastomeric organopolysiloxane comprising a polyoxyethylenated and/or polyoxypropylenated chain, for stabilizing a triple oil/water/oil emulsion.
